# EUROPÄISCHE PATENTSCHRIFT

(11) **EP 4 205 807 B1**
(45) Veröffentlichungstag und Bekanntmachung des Hinweises auf die Patenterteilung: **29.10.2025**
(21) Anmeldenummer: 21218312.3
(22) Anmeldetag: 30.12.2021
(51) Int. Cl.: A61N 5/06, A61F 13/00

(54) **WUNDAUFLAGE ZUR ANTIMIKROBIELLEN WUNDBEHANDLUNG**
WOUND DRESSING FOR ANTIMICROBIAL WOUND TREATMENT
PANSEMENT DESTINÉ AU TRAITEMENT ANTIMICROBIEN DES PLAIES

(43) Veröffentlichungstag der Anmeldung: 05.07.2023
(73) Patentinhaber: Paul Hartmann AG, 89522 Heidenheim (DE)
(72) Erfinder: DEIBLER, Martin, 89542 Herbrechtingen (DE); CROIZAT, Pierre, 89542 Herbrechtingen (DE)
(74) Vertreter: Paul Hartmann AG Patents & Licensing

(56) Entgegenhaltungen:
- WO-A2-2012/031282
- FR-A1- 2 986 156
- KR-A- 20080 018 360
- TW-U- M 586 146
- US-A1- 2018 280 723

## Beschreibung

Die vorliegende Erfindung betrifft eine Wundauflage zur Therapie von Wunden zur lokalen Therapie von mikrobiellen Kontaminationen, Infektionen und Biofilmen durch Plasmonenresonanz-induzierte thermische Mikroablation.

Mikrobielle Kontaminationen und Infektionen stellen eine Herausforderung in der Behandlung von Wunden, insbesondere in der Behandlung von chronischen Wunden, dar. Bei einer Therapie von infizierten Wunden mittels antimikrobieller Wirkstoffe können Resistenzen gegen die Wirkstoffe auftreten.

Im kosmetischen Bereich ist die Anwendung von Plasmonenresonanz bekannt. US2014/0371664 beschreibt eine Methode, bei der Nanopartikel auf einen kosmetischen Träger aufgebracht werden und in Hautnähe zur Plasmonenresonanz angeregt werden. Die Methode kann zur Haarentfernung und Akne-Behandlung eingesetzt werden.

Im Bereich der Wundbehandlung ist die Anwendung von Nanopartikeln zur antibakteriellen bzw. bakteriostatischen Therapie in WO2017/122224 beschrieben. Hierzu werden Silbernanopartikel mit Cellulose-Nanokristallen in einer Salbe kombiniert.

In WO2012/031282 werden Nanopartikel, welche in Fibroin eingebracht sind, zur Plasmonenresonanz angeregt. Der resultierende photothermische Effekt kann in der Thermotherapie genutzt werden.

FR2 986 156 betrifft ein transdermales Pflaster zur Anwendung auf der Hautoberfläche oder der Schleimhautmembran, in dessen Substrat Wirkstoff über eine photolabile Bindung gebunden ist. Durch einen Lichtimpuls im ultravioletten Bereich wird der Wirkstoff freigesetzt. Der Lichtimpuls wird von einer LED erzeugt, die gegenüber der Kante des Substrats angeordnet ist.

TW M 586 146 D2 offenbart eine Wundauflage mit Nanopartikeln aus Gold in einer Heizlage, welche zur Plasmonenresonanz angeregt werden können. Die durch die Plasmonenresonanz entstehende Wärme wird dazu verwendet, dass ein Wirkstoff gezielt an die Wunde abgegeben werden kann.

US 2018/289723 betrifft die Photoeradikation von Mikroorganismen mittels einer flexiblen Lichtquelle. Das Ziel, bei dem es sich um eine Wunde handeln kann, wird mit blauem und violettem Licht bestrahlt. Die Lichtquelle umfasst Elektroden und Lichtemitter. Der Lichtemitter befindet sich zwischen zwei Elektroden. Mindestens die hautzugewandte Elektrode muss transparent sein. Die transparenten Elektroden können neben Graphen auch Nanopartikel enthalten, mittels welcher Oberflächenplasmonen angeregt werden können.

Die der Erfindung zugrunde liegende Aufgabe besteht darin, bei einer Wundbehandlung das Risiko für Wundinfektionen zu verringern. Eine weitere Aufgabe besteht darin, die mikrobielle Besiedlung von bereits infizierten Wunden zu reduzieren. Des Weiteren soll die Ausbildung von Biofilmen in Wunden unterdrückt und bereits vorhandene Biofilme sollen möglichst wirksam eliminiert werden.

Diese Aufgaben werden durch eine Wundauflage gemäß Anspruch 1 gelöst. Die Erfindung ermöglicht die Verbesserung der Therapie chronischer, insbesondere infizierter Wunden durch eine thermische Inaktivierung und gegebenenfalls Abtragung von Mikroorganismen und Biofilmen. Diese Hitzebehandlung kann zielgenau auf der Wundstelle oder gegebenenfalls in der Wundumgebung angewandt werden.

Die Wundauflage umfasst eine bei Gebrauch der Wundauflage wundseitig angeordnete Wundkontaktschicht, eine bei Gebrauch der Wundauflage wundabseitig angeordnete Abdeckschicht und eine Lichtquelle, die zwischen Wundkontaktschicht und Abdeckschicht angeordnet ist.

Die erfindungsgemäße Wundauflage ist weiter dadurch gekennzeichnet, dass in der Wundkontaktschicht Nanopartikel vorhanden sind. Diese Nanopartikel sind geeignet zu Plasmonenresonanz angeregt zu werden. Die Lichtquelle ist dazu geeignet, die Nanopartikel zur Plasmonenresonanz anzuregen.

Mittels der erfindungsgemäßen Wundauflage kann somit über einem Wundbereich ebenso wie über der in der Wundumgebung vorhandenen unversehrten Haut eines Patienten eine Plasmonenresonanz angeregt werden. Die daraus resultierende thermische Mikroablation führt zur Inaktivierung und/oder Abtragung von schädlichen Mikroorganismen oder Biofilmen in einer Wunde oder auf der Haut eines Patienten. Unter einer thermischen Mikroablation wird vorliegende thermische Inaktivierung und gegebenenfalls Abtragung von Mikroorganismen und Biofilmen verstanden.

Durch die Wechselwirkung der in der Wundkontaktschicht vorhandenen Nanopartikel mit Licht werden die Leitungselektronen in den Nanopartikeln zur Schwingung angeregt. Das Quasiteilchen, das diese Schwingung beschreibt, ist das Plasmon. Die Anregung eines Plasmons geht mit der Absorption von Licht einher und wird als Plasmonenresonanz bezeichnet.

Unter Nanopartikeln werden im Zusammenhang mit der vorliegenden Erfindung Teilchen mit einer maximalen räumlichen Ausdehnung von 1 nm bis 500 nm verstanden. Bevorzugt liegt die maximale räumliche Ausdehnung der Nanopartikel zwischen 2 nm und 200 nm, besonders bevorzugt liegt die maximale räumliche Ausdehnung von Nanopartikeln zwischen 5 nm und 150 nm, insbesondere liegt die maximale räumliche Ausdehnung von Nanopartikeln zwischen 6 nm und 100 nm.

Die maximale räumliche Ausdehnung der Nanopartikel wird im Zusammenhang mit der Erfindung vorzugsweise mittels Rasterelektronenmikroskopie bestimmt.

Die maximale Ausdehnung der Nanopartikel unterliegt einer Verteilung und kann daher von Nanopartikel zu Nanopartikel um wenige Nanometer schwanken.

Gemäß der vorliegenden Erfindung werden Nanopartikel in eine Wundkontaktschicht eingebracht oder auf eine Oberfläche der Wundkontaktschicht aufgebracht. Durch die Bestrahlung mit Licht werden diese Nanopartikel zu Schwingungen angeregt. Durch die oben beschriebene Plasmonenresonanz kann lokal im Wundbereich oder in der Wundumgebung Wärme erzeugt werden. Durch thermische Mikroablation können schädliche Mikroorganismen oder Biofilme von der Wunde oder der Haut eines Patienten entfernt werden.

Die Nanopartikel können beispielsweise in eine Silikonmatrix eingebracht sein. Auch können die Nanopartikel auf die Wundkontaktschicht aufgebracht sein, beispielsweise mittels Tauchbeschichtung oder mittels Lösungsmittelverdampfung. Bei der Lösungsmittelverdampfung werden die Nanopartikel in einem Lösungsmittel vermengt. Das Lösungsmittel wird bei reduziertem Druck entfernt, wobei die Nanopartikel ausfallen und auf der Oberfläche der Wundkontaktschicht haften.

Die Abdeckschicht soll im Wundbereich ein für die Wundheilung förderliches Milieu schaffen und verhindern, dass Verunreinigungen in die Wunde gelangen. Die Abdeckschicht kann ein integraler Teil der Wundauflage sein. Alternativ kann die Abdeckschicht als separate Komponente bereitgestellt werden und erst bei Gebrauch der Wundauflage aufgebracht werden. Die Abdeckschicht kann selbstklebend ausgeführt sein.

Die Wundauflage umfasst eine Wundkontaktschicht. Die Wundkontaktschicht kommt bei Anwendung der Wundauflage direkt auf der Haut oder Wundstelle des Patienten zum Liegen.

Die Wundkontaktschicht kann einen adhäsiven Bereich zum Befestigen der Wundauflage aufweisen.

Bevorzugt umfasst die Wundauflage eine Zwischenschicht. Die Zwischenschicht ist zwischen Abdeckschicht und Wundkontaktschicht angeordnet. Die Zwischenschicht umfasst bevorzugt ein Flüssigkeits-absorbierendes Material, wie beispielsweise Zellulosefasern, Baumwollfasern, Viscosefasern, Alginatfasern, hydrophile synthetische Polymere oder superabsorbierende Partikel oder Fasern. Das Material kann beispielsweise in Form eines Non-Woven, Polymerschaums oder Hydrogels vorhanden sein. Vorzugsweise enthält die Zwischenschicht ein Vlies oder Airlaid, in welches superabsorbierende Partikel oder Fasern aus Polyacrylat eingearbeitet sind. Es können auch mehrere Zwischenschichten, insbesondere aus unterschiedlichen Materialien, vorhanden sein.

Bei den einen oder mehreren Zwischenschichten kann es sich um eine oder mehrere Transferschichten handeln. Eine Transferschicht ermöglicht insbesondere einen unidirektionalen Transport von aus der Wunde abgegebenem Wundsekret. Bevorzugte Ausführungsformen einer solchen Transferschicht umfassen Materialien, die Kapillareffekte zulassen oder hygroskopische Eigenschaften aufweisen, wie beispielsweise Fleece, superabsorbierende Fasern oder Partikel oder dreidimensional perforierte Folien.

Bei den Nanopartikeln handelt es sich in einer bevorzugten Ausführungsform um Nanostäbchen. Diese weisen insbesondere eine maximale räumliche Ausdehnung von 45 nm auf. Die Nanopartikel können gleichfalls in weiteren Formen vorliegen, z.B. Kubus, Kugel, Tetrahedron, Octahedron, Plättchen oder Röhrchen. Eine mögliche Ausführungsform der Nanopartikel kann Nanoröhrchen, Nanostäbchen, Nanofasern, aber auch Nanoplättchen oder hohle geschlossene Moleküle umfassen. Beispiele für solche Ausführungsformen sind Goldnanostäbchen oder Goldnanohüllen um einen Siliziumoxidkern.

Vorzugweise umfassen die Nanopartikel metallische Ausgangssubstanzen oder metallische Salze und Oxide. Besonders bevorzugt bestehen die Nanopartikel aus den vorgenannten metallischen Ausgangssubstanzen oder metallischen Salzen und Oxiden. Gemäß einer besonders vorteilhaften Ausführungsform umfassen die Nanopartikel Gold oder Zinkoxid oder bestehen ausschließlich daraus.

Nanopartikel können auch Silber, Ruthenium, Platin, Rhodium, Osmium, Iridium, Kupfer, Zink, Nickel, Chrom, Magnesium, Eisen, Palladium, Gold, Titan, Titandioxide, Silber, Silbernitrat und weiter Silber-Derivate umfassen.

In einer anderen bevorzugten Ausführungsform umfasst die Wundauflage mindestens eine Zwischenschicht und mindestens eine Lichtquelle. Die mindestens eine Lichtquelle ist bei dieser Ausführungsform auf der von der Wunde abgewandten Seite der mindestens einen Zwischenschicht angebracht. Die Zwischenschicht umfasst einen transparenten Bereich und/oder eine Aussparung, so dass ein lichtdurchlässiger Bereich zwischen der mindestens einen Lichtquelle und der Wundkontaktschicht entsteht. Das von der Lichtquelle abgestrahlte Licht dringt durch die transparenten Bereiche und/oder Aussparungen bis zur Wundkontaktschicht vor und regt die in die Wundkontaktschicht eingebrachten Nanopartikel zur Plasmonenresonanz an.

Um eine ausreichende Bestrahlung der Nanopartikel zu gewährleisten, umfassen generell alle Schichten, die zwischen Lichtquelle und Wundkontaktschicht angeordnet sind (falls vorhanden) einen transparenten Bereich und/oder eine Aussparung. Die transparenten Bereiche und/oder Aussparungen sind so übereinander angeordnet, dass ein lichtdurchlässiger Bereich zwischen der Lichtquelle und der Wundkontaktschicht vorhanden ist.

In einer weiteren, besonders bevorzugten Ausführungsform ist die Lichtquelle zwischen der Wundkontaktschicht und der Zwischenschicht so angeordnet, dass die Lichtquelle direkt auf der Wundkontaktschicht aufliegt. Diese Ausführungsform hat den Vorteil, dass das Licht auf dem Weg von der Lichtquelle zur Wundkontaktschicht nur wenig Intensität verliert. Intensitätsverluste können beispielsweise durch Absorption oder durch Reflektionen an Grenzflächen auftreten.

Bei der Lichtquelle handelt es sich bevorzugt um eine Laser-LED oder eine LED. Es kann sich auch um eine Anordnung von mehreren Laser-LEDs oder LEDs in einem Array handeln.

Falls zur Plasmonenanregung ein Laser eingesetzt wird, darf der Laser eine Flächenleistung von 330 bis 350 mW/cm² für 10 bis 100 s nicht überschreiten, sonst kann es zu Zell- und Hautschäden kommen. Es wird bevorzugt der Wellenlängenbereich 650 nm bis 940 nm im Nahinfrarotbereich und/oder 1000 nm bis 1350 nm im Infrarotbereich verwendet.

Die Lichtquelle, die zur Erzeugung der Plasmonenresonanz verwendet wird, strahlt Licht im Wellenlängen-Bereich zwischen 300 nm und 2000 nm aus.

Bevorzugt strahlt die Lichtquelle monochromatisches Licht aus, beispielsweise Licht mit einer Wellenlänge von 420 nm. Bevorzugt strahlt die Lichtquelle monochromatisches Licht aus, welches auf die Größe und Form der Nanopartikel abgestimmt ist, so dass PlasmonenResonanz auftritt. Gemäß einer anderen bevorzugten Ausführungsform wird eine multimodale Lichtquelle verwendet. Die multimodale Lichtquelle strahlt Licht mit unterschiedlichen diskreten Frequenzen ab.

Generell sollte bei einer Bestrahlung von 10 bis 100 s das von der Lichtquelle ausgestrahlte Licht 350 mW/cm² nicht überschreiten, da sonst beim Patienten Zell- oder Hautschäden auftreten können.

Als Lichtquelle eignet sich beispielsweise eine LED mit einer Leistung von 900 mW bei 850 nm Nominalwellenlänge.

Es wird bevorzugt entweder eine Laser-LED oder eine LED eingesetzt. Auch LEDs in einem Array sind eine mögliche Ausführungsform für eine in die Wundauflage integrierte Lichtquelle.

Zur Stromversorgung der Lichtquelle können Leiter, beispielsweise leitfähige Fäden, eingesetzt werden. Die Leiter werden bevorzugt unter der Abdeckschicht herausgeführt und mit einem Anschluss versehen, so dass eine Stromquelle an die Wundauflage angeschlossen werden kann.

Die Lichtquelle kann über ein integriertes Stromversorgungselement verfügen. Das Stromversorgungselement kann eine Dünnschicht-Energiequelle wie beispielsweise eine flexible Batterie sein. Flexible Batterien können beispielsweise durch J.Flex flexible Lithium Polymer Batterien, Panasonic flexible Lithium-Ion Batterien, GrePow flexible high discharge LiFePO4 Batterien, flexible Lithium-Sulfur Batterien oder Sodium-Ionen Batterien (SIBs) realisiert werden.

Die Stromversorgung der Lichtquelle erfolgt vorzugsweise über externe Energiequellen und kontaktlosem Energietransfer mittels elektromagnetischer Felder. In einer Ausführungsform ist das Stromversorgungselement ein Energy-Harvesting-Element. Das Energy-Harvesting-Element nutzt bevorzugt elektromagnetische Strahlung als Energiequelle. Besonders bevorzugt sind dabei Radiowellen mit Frequenzen von 30 kHz bis zu 300 GHz. Diese Radiowellen können beispielsweise von Funktürmen, Radaranlagen, WiFi-Netzen oder Bluetooth-Verbindungen stammen. Für WiFi Netze sind Radiowellen mit Frequenzen von 2,4 GHz und einer Leistung bis 100 mW, sowie mit Frequenzen von 5 GHz und einer Leistung bis 1 W von besonderem Interesse. Auf Mobilfunknetzen/GSM beruhende Radiowellen haben nach den GSM-Standards die Frequenzen 850 MHz, 900 MHz, 1800 MHz und 1900 MHz.

In einer bevorzugten Ausführungsform umfasst die Stromversorgung der Lichtquelle ein Energiespeichermodul.

In einer anderen bevorzugten Ausführungsform wird die Lichtquelle über induktives Laden mit Energie versorgt.

Die elektronischen Bauteile der erfindungsgemäßen Wundauflage sind bevorzugt teilelastisch oder voll-elastisch ausgebildet. Die Laser-LEDs eines Laser-LED Arrays sind auf einem flexiblen Träger aufgebracht oder eingebettet und werden durch flexible elektrische Leiter kontaktiert. Der flexible Träger kann eine Silikonschicht sein.

Nach einer Ausführungsform umfasst die Wundauflage mindestens zwei Lichtquellen. Die Lichtquellen sind einzeln ansteuerbar, d.h. die Aktivierung einer ersten Lichtquelle erfolgt unabhängig von der Aktivierung einer zweiten Lichtquelle.

Insofern eine Vielzahl von Lichtquellen vorgesehen ist, werden bevorzugt diejenigen Lichtquellen aktiviert, welche bei Gebrauch der Wundauflage über einem Wundbereich vorhanden sind, während diejenigen Lichtquellen, welche über unversehrten Hautbereichen vorhanden sind, deaktiviert sind. So kann die Behandlung lokal auf die Wundfläche beschränkt werden.

Die Wundfläche kann über Sensoren ermittelt werden, die in die Wundauflage integriert sind. Bevorzugt wird ein periodische Gesamtanordnung von mehreren Sensoren verwendet. Die periodische Gesamtanordnung kann beispielsweise die Anordnung von Sensoren in einer Matrix sein.

In einer anderen Ausführungsform wird eine erste Abbildung des zu behandelnden Wundbereichs von einer Kamera aufgenommen. Die erste Abbildung wird dann mittels eines digitalen Bildauswertungsverfahrens segmentiert, so dass die ein oder mehreren Segmente der ersten Abbildung, die die Wunde darstellen, als Wundfläche gekennzeichnet werden. Nachfolgend wird eine erfindungsgemäße Wundauflage auf den zu behandelnden Wundbereichs appliziert. Sodann erfolgt eine zweite Abbildung des zu behandelnden Wundbereichs einschließlich der aufgelegten Wundauflage. Die erste Abbildung wird unter Einsatz eines digitalen Verfahrens mit der zweiten Abbildung zur Deckung gebracht. Dies kann beispielsweise mittels in beiden Abbildungen vorkommenden Punkten erfolgen. Der erfolgreiche Abgleich der beiden Abbildungen ermöglicht es sodann, die in der zweiten Abbildung festgehaltene Position der aufgebrachten Wundauflage relativ zur der anhand der ersten Abbildung ermittelten Wundfläche zu bestimmen. Anhand der Positionsinformation können nun genau diejenigen Lichtquellen, welche über der Wundfläche liegen, gezielt aktiviert werden.

### Abbildung 1

Schematische Darstellung einer Ausführungsform der Wundauflage mit integrierter Lichtquelle

### Abbildung 2

Schematische Darstellung einer weiteren Ausführungsform der Wundauflage mit integrierter Lichtquelle

### Abbildung 3

Schematische Darstellung einer Zwischenschicht, welche eine Vielzahl von periodisch angeordneten Lichtquellen umfasst

Alle lichtdurchlässigen Schichten 30 sind gepunktet dargestellt.

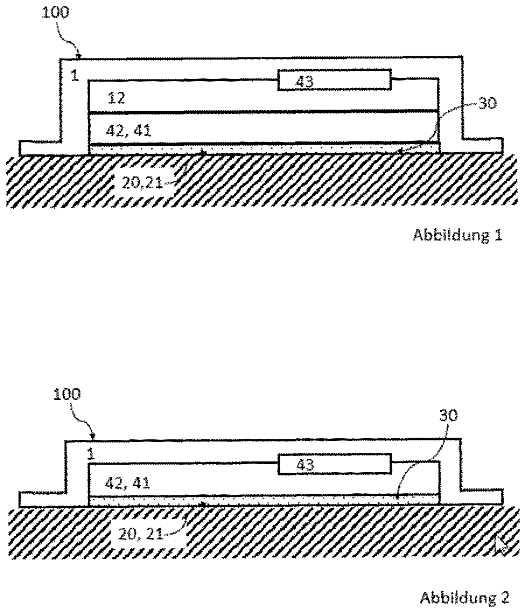

Abbildung 1 zeigt eine bevorzugte Ausführungsform der Wundauflage 100. Die Wundauflage 100 umfasst eine Abdeckschicht 1, eine Wundkontaktschicht 20 und eine absorbierende Zwischenschicht 12, wobei die Zwischenschicht 12 zwischen Abdeckschicht 1 und Wundkontaktschicht 20 angeordnet ist. Die Wundkontaktschicht 20 enthält Nanostäbchen 21 aus Gold, wobei vorteilhaft die in der Veröffentlichung "Bacterial biofilm elimination using gold nanorod localised surface plasmon resonance generated heat" von Maria Pihlet al. (2017) (https://doi.org/10.1016/j.msec.2017.05.067.) offenbarten Goldpartikel eingesetzt werden können. Die maximale räumliche Ausdehnung der Nanostäbchen 21 beträgt 45 nm. Die Wundkontaktschicht 20 liegt bei Gebrauch der Wundauflage direkt auf der Haut und/oder der Wundstelle des Patienten auf.

Die Wundauflage 100 umfasst eine Schicht 42 mit einer integrierten Lichtquelle 41. Bei der Lichtquelle 41 kann es sich um ein Laser-LED Array handeln. Die Lichtquelle 41 ist eine LED mit 850 nm Nominalwellenlänge, sowie 3,5 mW/cm² bei 322 mW Gesamtleistung.

Die Schicht 42 ist auf der wundzugewandten Seite der Zwischenschicht 12 aufgebracht. Die Schicht 42 umfasst ein Laser-LED Array, welches auf einen Träger mit einer Netzstruktur aufgebracht ist. Aufgrund der Netzstruktur des Trägers weist die Schicht 42 eine Vielzahl von Durchgängen auf, so dass Wundexsudat von der Wunde zur Zwischenschicht gelangen kann. Die Zwischenschicht 12 umfasst Mikrofasern, die eine hohe Absorptionsfähigkeit für Flüssigkeiten aufweisen. In der Zwischenschicht 12 kann deshalb in Wundexsudat dispergierte Zell- und Baktierendebris, die durch thermische Mikroablation von der Wunde gelöst wurde, angesaugt und gebunden werden.

Das vom Laser-LED Array erzeugte Licht trifft ungehindert auf die Nanostäbchen 21 in der Wundkontaktschicht 20 und regt diese zur Plasmonenresonanz an. Die daraus resultierende lokale Erwärmung führt zu einer thermischen Mikroablation und/oder einer Inaktivierung von schädlichen Mikroorganismen oder Biofilmen in einer Wunde eines Patienten.

Die Energieversorgung 43 des Laser-LED Arrays erfolgt über ein Energy-Harvesting-Element. Das Energy-Harvesting-Element kann die in der Umgebung zur Verfügung stehende Energie in elektrische Energie umwandeln. Das Energy-Harvesting-Element nutzt bevorzugt elektromagnetische Strahlung mit Frequenzen von 2,4 GHz und einer Leistung bis 100 mW, sowie mit Frequenzen von 5 GHz und einer Leistung bis 1 W, als Energiequelle.

Die Wundauflage 100 wird am Patienten aufgebracht, so dass die Wundkontaktschicht über der Wundfläche zum Liegen kommen. Die Lichtquelle 41 wird eingeschaltet und die Nanostäbchen 21 aus Gold werden zur Plasmonenresonanz angeregt. Die Lichtquelle 41 bleibt 5 min aktiv. Je nach Bedarf kann die Lichtquelle 41 mehrmals im Laufe der Behandlung aktiviert werden.

In Abbildung 2 wird eine weitere bevorzugte Ausführungsform der Wundauflage 100 schematisch dargestellt. Die Wundauflage umfasst eine Abdeckschicht 1 und eine Wundkontaktschicht 20, welche Nanostäbchen 21 aus Silber enthält. Die Nanostäbchen 21 sind in lichtdurchlässiges Silikon eingebettet. Die Nanostäbchen werden über eine integrierte Lichtquelle 41, wie ein Laser-LED Array, zur Plasmonenresonanz angeregt. Das Laser-LED Array ist in eine Zwischenschicht 42 integriert. Das Laser-LED Array 41 wird über die Energieversorgung 43 mit Energie versorgt. Die Energieversorgung 43 ist eine flexible Batterie, wie beispielsweise Panasonic flexible Lithium-Ion Batterien. Die Wundkontaktschicht 20 ist lichtdurchlässig.

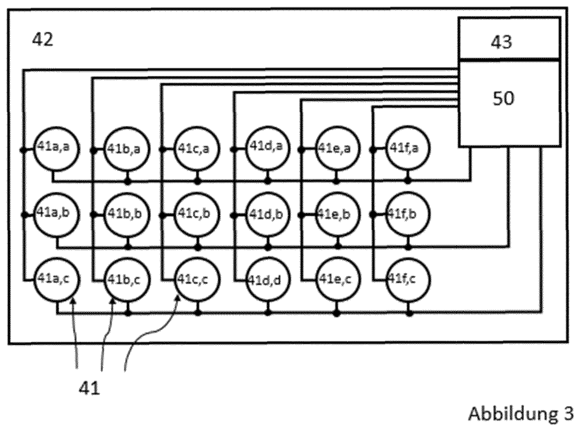

Abbildung 3 zeigt eine schematische Darstellung einer Zwischenschicht 42 mit mehreren integrierten Lichtquellen 41. Die Lichtquellen 41a,a bis 41f,c können einzeln durch das Steuerungselement 50 aktiviert werden. Das Steuerungselement 50 umfasst einen Mikrocontroller und ein Empfängerelement. Mittels des Empfängerelements erhält das Steuerungselement Aktivierungsanweisungen von einer externen Berechnungseinheit, die nicht Teil der Wundauflage ist. Eine Aktivierungsanweisung kann der Befehl sein, alle Lichtquellen aus 41a,a bis 41f,c zu aktivieren, die über der Wundfläche liegen. Um welche Lichtquellen aus 41a,a bis 41f,c es sich dabei handelt, wird mittels der externen Berechnungseinheit bestimmt. Durch eine flexible Batterie 43 wird das Steuerungselement 50 und die Lichtquellen 41 mit Energie versorgt.

## Patentansprüche

1. Wundauflage (100) zur Verwendung bei der Inaktivierung und/oder Abtragung von schädlichen Mikroorganismen oder Biofilmen in einer Wunde eines Patienten mittels Plasmonenresonanz-induzierter thermischer Mikroablation, umfassend:
- eine wundseitig angeordnete Wundkontaktschicht (20),
- eine wundabseitig angeordnete Abdeckschicht (1),
- eine Lichtquelle (41), die zwischen Wundkontaktschicht (20) und Abdeckschicht (1) angeordnet ist,
**dadurch gekennzeichnet,**
**dass** die Wundkontaktschicht (20) Nanopartikel (21) enthält, die geeignet sind zu Plasmonenresonanz angeregt zu werden, und dass die Lichtquelle (41) dazu geeignet ist, die Nanopartikel (21) zur Plasmonenresonanz anzuregen.

2. Wundauflage (100) nach Anspruch 1, wobei die Wundkontaktschicht (20) transparente Bereiche umfasst.

3. Wundauflage (100) nach Anspruch 1 oder 2, wobei die Wundauflage (100) mindestens eine Zwischenschicht (12) umfasst und die mindestens eine Zwischenschicht (12) zwischen der Abdeckschicht (1) und der Wundkontaktschicht (20) angeordnet ist.

4. Wundauflage (100) nach Anspruch 3, wobei alle Schichten, die zwischen Lichtquelle (41) und Wundkontaktschicht (20) angeordnet sind, einen transparenten Bereich (30) und/oder eine Aussparung umfassen, so dass ein lichtdurchlässiger Bereich (30) zwischen der Lichtquelle (41) und der Wundkontaktschicht (20) vorhanden ist.

5. Wundauflage (100) nach einem der Ansprüche 1 bis 3, wobei die Lichtquelle (41) wundabseitig unmittelbar auf der Wundkontaktschicht (20) aufliegt.

6. Wundauflage (100) nach einem der Ansprüche 3 bis 5, wobei die mindestens eine Zwischenschicht (12) Flüssigkeits-absorbierende Eigenschaften aufweist.

7. Wundauflage (100) nach mindestens einem der vorgenannten Ansprüche, wobei die Wundkontaktschicht (20) einen adhäsiven Bereich aufweist.

8. Wundauflage (100) nach mindestens einem der vorgenannten Ansprüche, wobei es sich bei den Nanopartikeln (21) um Nanopartikel (21) aus Metall oder metallischen Salzen handelt.

9. Wundauflage (100) nach mindestens einem der vorgenannten Ansprüche, wobei die Nanopartikel (21) eine maximale räumliche Ausdehnung zwischen 1 nm und 500 nm, bevorzugt zwischen 2 nm und 200 nm, besonders bevorzugt zwischen 5 nm und 150 nm, insbesondere zwischen 6 nm und 100 nm haben.

10. Wundauflage (100) nach mindestens einem der vorgenannten Ansprüche, wobei es sich bei der Lichtquelle (41) insbesondere um eine LED handelt.

11. Wundauflage (100) nach mindesten einem der vorgenannten Ansprüche, wobei die Wundauflage mindestens zwei Lichtquellen (41) umfasst.

12. Wundauflage (100) nach Anspruch 11, wobei die Lichtquellen (41) einzeln angesteuert werden können und wobei nur die Lichtquellen (41) der mindestens zwei Lichtquellen (41) aktiviert werden, die sich über der Wundfläche befinden.

## Claims

1. Wound dressing (100) for use in the inactivation and/or removal of harmful microorganisms or biofilms in a wound of a patient by means of plasmon resonance-induced thermal microablation, comprising:
- a wound contact layer (20) arranged on the wound side,
- a cover layer (1) arranged on the non-wound side,
- a light source (41) arranged between wound contact layer (20) and cover layer (1),
**characterized**
**in that** the wound contact layer (20) contains nanoparticles (21) which are suitable for excitation to plasmon resonance, and in that the light source (41) is suitable for exciting the nanoparticles (21) to plasmon resonance.

2. Wound dressing (100) according to Claim 1, wherein the wound contact layer (20) comprises transparent regions.

3. Wound dressing (100) according to Claim 1 or 2, wherein the wound dressing (100) comprises at least one intermediate layer (12) and the at least one intermediate layer (12) is arranged between the cover layer (1) and the wound contact layer (20).

4. Wound dressing (100) according to Claim 3, wherein all the layers which are arranged between light source (41) and wound contact layer (20) comprise a transparent region (30) and/or a cutout, so that a light-permeable region (30) is present between the light source (41) and the wound contact layer (20).

5. Wound dressing (100) according to any of Claims 1 to 3, wherein the light source (41) on the non-wound side lies directly on the wound contact layer (20).

6. Wound dressing (100) according to any of Claims 3 to 5, wherein the at least one intermediate layer (12) has fluid-absorbing properties.

7. Wound dressing (100) according to at least one of the preceding claims, wherein the wound contact layer (20) has an adhesive region.

8. Wound dressing (100) according to at least one of the preceding claims, wherein the nanoparticles (21) are nanoparticles (21) composed of metal or metallic salts.

9. Wound dressing (100) according to at least one of the preceding claims, wherein the nanoparticles (21) have a maximum spatial extent of between 1 nm and 500 nm, preferably between 2 nm and 200 nm, more preferably between 5 nm and 150 nm, more particularly between 6 nm and 100 nm.

10. Wound dressing (100) according to at least one of the preceding claims, wherein the light source (41) more particularly is an LED.

11. Wound dressing (100) according to at least one of the preceding claims, wherein the wound dressing comprises at least two light sources (41).

12. Wound dressing (100) according to Claim 11, wherein the light sources (41) can be driven individually and wherein only those light sources (41) of the at least two light sources (41) that are located over the wound surface are activated.

## Revendications

1. Pansement (100) destiné à être utilisé lors de l'inactivation et/ou de l'enlèvement de micro-organismes ou de biofilms nuisibles dans une plaie d'un patient au moyen d'une microablation thermique induite par résonance plasmonique, comprenant :
- une couche de contact (20) avec la plaie, agencée côté plaie,
- une couche de recouvrement (1) agencée sur le côté opposé à la plaie,
- une source de lumière (41), qui est agencée entre la couche de contact (20) avec la plaie et la couche de recouvrement (1),
**caractérisé**
**en ce que** la couche de contact (20) avec la plaie contient des nanoparticules (21) qui conviennent pour être excitées en résonance plasmonique et **en ce que** la source de lumière (41) convient pour exciter les nanoparticules (21) en résonance plasmonique.

2. Pansement (100) selon la revendication 1, la couche de contact (20) avec la plaie comprenant des zones transparentes.

3. Pansement (100) selon la revendication 1 ou 2, le pansement (100) comprenant au moins une couche intermédiaire (12) et ladite au moins une couche intermédiaire (12) étant agencée entre la couche de recouvrement (1) et la couche de contact (20) avec la plaie.

4. Pansement (100) selon la revendication 3, toutes les couches qui sont agencées entre la source de lumière (41) et la couche de contact (20) avec la plaie comprenant une zone (30) transparente et/ou un évidement de telle sorte qu'il existe une zone (30) transparente à la lumière entre la source de lumière (41) et la couche de contact (20) avec la plaie.

5. Pansement (100) selon l'une des revendications 1 à 3, la source de lumière (41) se situant, côté plaie, directement sur la couche de contact (20) avec la plaie.

6. Pansement (100) selon l'une des revendications 3 à 5, ladite au moins une couche intermédiaire (12) présentant des propriétés d'absorption de liquide.

7. Pansement (100) selon au moins l'une des revendications susmentionnées, la couche de contact (20) avec la plaie présentant une zone adhésive.

8. Pansement (100) selon au moins l'une des revendications susmentionnées, les nanoparticules (21) étant des nanoparticules (21) en métal ou en sels métalliques.

9. Pansement (100) selon au moins l'une des revendications susmentionnées, les nanoparticules (21) présentant une étendue spatiale maximale entre 1 nm et 500 nm, de préférence entre 2 nm et 200 nm, de manière particulièrement préférée entre 5 nm et 150 nm, en particulier entre 6 nm et 100 nm.

10. Pansement (100) selon au moins l'une des revendications susmentionnées, la source de lumière (41) étant en particulier une DEL.

11. Pansement (100) selon au moins l'une des revendications susmentionnées, le pansement comprenant au moins deux sources de lumière (41).

12. Pansement (100) selon la revendication 11, les sources de lumière (41) pouvant être commandées individuellement et seules les sources de lumière (41) parmi les au moins deux sources de lumière (41) qui se trouvent au-dessus de la surface de la plaie étant activées.
